(19) Europäisches Patentamt
European Patent Office
Office européen des brevets

(11) **EP 2 602 025 A1**

(12) **EUROPEAN PATENT APPLICATION**

(43) Date of publication:
**12.06.2013 Bulletin 2013/24**

(51) Int Cl.:
***B01L 3/00*** (2006.01)

(21) Application number: **12195366.5**

(22) Date of filing: **04.12.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME** | (72) Inventors:<br>• **Kim, Seung Hoon**<br>  **Gyeonggi-do (KR)**<br>• **Lee, Yong Koo**<br>  **Gyeonggi-do (KR)** |
| (30) Priority: **05.12.2011 KR 20110129236** | (74) Representative: **Grünecker, Kinkeldey, Stockmair & Schwanhäusser**<br>**Leopoldstrasse 4**<br>**80802 München (DE)** |
| (71) Applicant: **Samsung Electronics Co., Ltd**<br>**Gyeonggi-do 443-742 (KR)** | |

(54) **Microfluidic device and microfluidic system including the same**

(57)     A microfluidic device capable of detecting whether a test is conducted as designed using a single chamber, and a microfluidic system including the same are provided. The microfluidic device includes a platform, a plurality of chambers disposed in the platform and configured to contain a fluid, and at least one channel connecting the chambers, wherein at least one of the chambers comprises a first container and a second container, a depth of the first container is greater than a depth of the second container, and a cross-sectional area of the first container is different from a cross-sectional area of the second container.

FIG. 4

**Description**

BACKGROUND

1. Field

Apparatuses and systems consistent with exemplary embodiments relate to a microfluidic device configured to measure absorbance differences of fluids using a single chamber and a microfluidic system including the same.

2. Description of the Related Art

Transferring a fluid within a microfluidic device may require a drive pressure, such as capillary pressure or pressure generated using a separate pump. In recent years, disc-shaped microfluidic devices in which microfluidic structures are disposed in a disc-shaped body to enable flowing of a fluid using centrifugal force have been suggested to conduct a series of operations. These are referred to as Lab Compact Disk (CD), Lab on a disk, or Digital Bio Disk (DBD).

Generally, a disc-shaped microfluidic device includes a chamber to contain a fluid, a channel through which the fluid flows, and a valve to control the flow of the fluid, and may be manufactured by various combinations thereof.

A microfluidic device may be used as a sample test device to analyze a sample such as blood, saliva, and urine. A reagent reacting with specific substances contained in the sample may be disposed in the microfluidic device. Thus, a sample may be tested by injecting the sample into the microfluidic device and observing the results of the reaction between the sample and the reagent.

To ensure reliability of a test performed using a microfluidic device, a quality check is required to confirm whether the test was performed as designed. By using three or more chambers, quantitative injection of the reagent may be determined to conduct the quality check. In this case, the configuration of the microfluidic device needs to be changed as the volume of the reagent varies. In addition, when a plurality of reagents are used, chambers for checking whether each of the reagents is properly injected are required.

SUMMARY

Exemplary embodiments provide a microfluidic device configured to whether a test is conducted as designed using a single chamber and a plurality of reagents, and a microfluidic system including the same.

In accordance with an aspect of an exemplary embodiment, there is provided a microfluidic device including a platform, a plurality of chambers disposed in the platform to contain a fluid, and at least one channel connecting the chambers. At least one of the plurality of chambers includes a plurality of containers that include at least a first container and a second container, wherein a depth of the first container is greater than a depth of the second container. A cross-sectional area of the first container is therefore different from a cross-sectional area of the second container. A distance between the first container and a central axis of the platform may be greater than a distance between the second container and the central axis of the platform.

When the same amount of fluid is injected into the plurality of containers, a height of the fluid in the first container may be different from a height of the fluid in the second container.

A boundary between the first container and the second container may be parallel to the bottom surface of the first container.

A boundary between the first container and the second container may include an inclined surface sloping upward from the first container toward the outer edge of the second container.

A boundary between the first container and the second container may include a declined surface sloping downward from the first container toward the outer edge of the second container.

The chambers may include a sample injection chamber to inject a fluid, one or more reaction chambers in which reactions of the fluid occur, and a quality check chamber to confirm whether a test is performed as designed.

The sample injection chamber may be disposed radially inward from the reaction chamber and the quality check chamber within the platform.

A cross-sectional area of the second container within the quality check chamber may be greater than a cross-sectional area of the first container within the quality check chamber.

A step difference may be formed at the center of the quality check chamber to form the first container and the second container.

The plurality of containers may further include a third container having a bottom surface that is higher than that of the second container.

A cross-sectional area of the third container may be different from a cross-sectional area of the second container, such that, when the same amount of fluid is injected into the containers, a height of the fluid in the third container is different from a height of the fluid in the second container.

The second container may be formed by forming a step difference at the center of the third container, and the first container may be formed by forming a step difference at the center of the second container.

The platform may be rotatable.

In accordance with an aspect of another exemplary embodiment, there is provided a microfluidic device includes a platform, a plurality of chambers disposed in the platform to contain a fluid, and at least one channel connecting the chambers. The chambers includes a sample injection chamber, at least one reaction chamber disposed radially outward of the sample injection chamber and a quality check chamber to confirm whether a test is performed properly. The quality check chamber may include a plurality of containers to contain a fluid. The containers may be formed by forming a step difference in an inner portion of the quality check chamber. The plurality of containers may include a first container and a second container, wherein a depth of the first container is greater than a depth of the second container. A cross-sectional area of the bottom surface of the second container may be greater than a cross-sectional area of the bottom surface of the first container.

A distance between the first container and a central axis of the platform may be greater than a distance between the second container and the central axis of the platform.

The bottom surface of the second container of the quality check chamber may include an inclined surface, sloping upward toward the outer edge of the quality check chamber.

The bottom surface of the second container of the quality check chamber may include an declined surface, sloping downward toward the outer edge of the quality check chamber.

The quality check chamber may be disposed at one end of a distribution channel connecting the quality check chamber with the reaction chamber, such that a fluid fills the quality check chamber after the reaction chamber is filled with the fluid.

In accordance with an aspect of another exemplary embodiment, there is provided a microfluidic system including a microfluidic device including a platform, a plurality of chambers to contain a fluid, and at least one channel connecting the chambers in which the fluid flows, a light source to irradiate optical energy onto the chambers, and an optical detector to measure absorbance of the fluid contained in the one or more chambers using optical energy passing through the chambers. At least one of the one or more chambers may include a plurality of containers that includes a first container and a second container, wherein a depth of the first container is greater than a depth of the second container. A cross-sectional area of the first container is different from a cross-sectional area of the second container.

A distance between the first container and a central axis of the platform may be greater than a distance between the second container and the central axis of the platform.

When the same amount of a fluid is injected into the plurality of containers, a height of the fluid in the first container may be different from a height of the fluid in the second container.

A boundary between the first container and the second container may include an inclined surface, sloping upward from the second container toward the outer edge of the first container.

A cross-sectional area of the second container may be greater than a cross-sectional area of the first container.

The microfluidic device may be disposed between the light source and the optical detector.

BRIEF DESCRIPTION OF THE DRAWINGS

The above and/or other aspects will become apparent and more readily appreciated from the following description of exemplary embodiments, taken in conjunction with the accompanying drawings of which:

FIG. 1 is a perspective view schematically illustrating a microfluidic device according to an exemplary embodiment;
FIG. 2 is an enlarged plan view of main components of a microfluidic device according to an exemplary embodiment;
FIG. 3 is an enlarged perspective view of main components of a microfluidic device according to an exemplary embodiment;
FIG. 4 is a view of a microfluidic device of an exemplary embodiment of illustrating the relationship between a quality check chamber and a central axis of a platform;
FIGS. 5A and 5B are cross-sectional views showing a quality check chamber of a microfluidic device according to exemplary embodiments taken along line A-A;
FIG. 6 is a cross-sectional view showing a quality check chamber of a microfluidic device according to another exemplary embodiment taken along line A-A;
FIG. 7 is a cross-sectional view showing a quality check chamber of a microfluidic device according to another exemplary embodiment taken along line A-A; and
FIG. 8 is a cross-sectional view showing a quality check chamber of a microfluidic device according to another exemplary embodiment taken along line A-A.

DETAILED DESCRIPTION

Reference will now be made in detail to exemplary embodiments o, examples of which are illustrated in the accompanying drawings, wherein like reference numerals refer to like elements throughout.

The configuration of a microfluidic device according to the exemplary embodiments described herein may be applied to various types of microfluidic devices. Herein, a microfluidic device including a sample chamber, a reaction chamber, and a quality check chamber will be described.

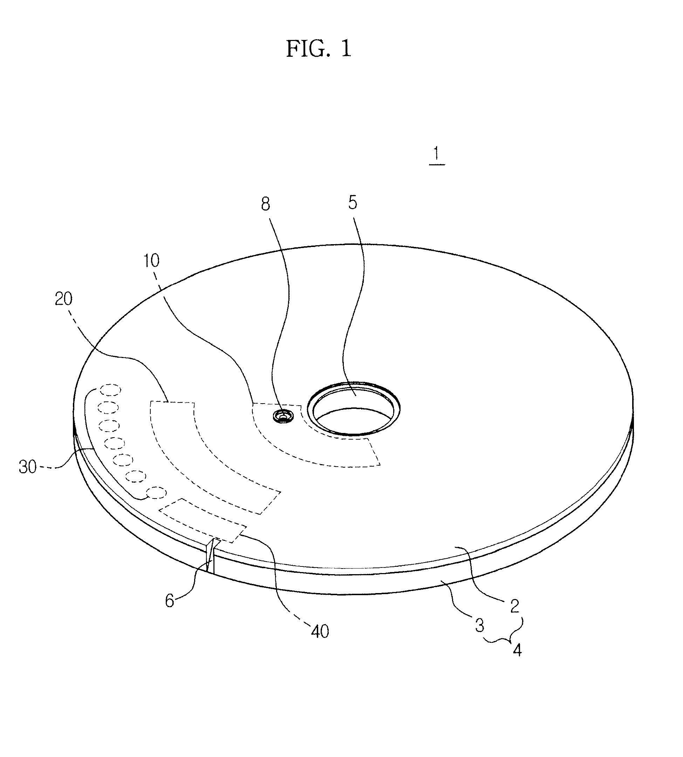

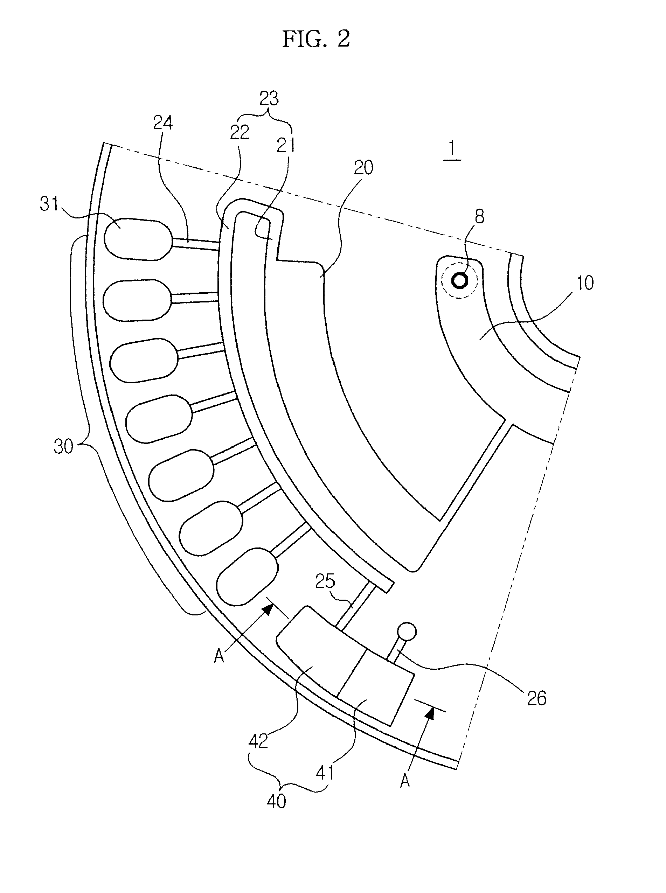

FIG. 1 is a perspective view schematically illustrating a microfluidic device 1 according to an exemplary embodiment. FIG. 2 is an enlarged plan view of main components of a microfluidic device 1 according to an exemplary embodiment.

Referring to FIGS. 1 and 2, the microfluidic device 1 includes a platform 4, one or more chambers provided in the platform 4 and containing a fluid, and one or more channels through which a fluid flows.

The platform 4 may be a rotatable disc-shaped platform and may rotate based on a central axis 5 of the platform 4. Action of the centrifugal force caused by rotation of the platform 4 enables movement of samples, centrifugal separation, mixing and the like in the chambers and channels provided in the platform 4.

The platform 4 is easily molded and the surface thereof may be made of a biologically inactive plastic such as acryl, PDMS, and PMMA. Any material may also be used for the platform 4 without limitation so long as it has chemical and biological stability, optical transparency, and mechanical processibility.

In an exemplary embodiment, the platform 4 may include a plurality of plates. Groove structures which correspond to the chambers, channels, and the like are formed on the surfaces of two of the plates contacting each other. Thus, when the plates are joined together, there is provided an area to contain a fluid within the platform 4, and a passage through which a fluid flows.

For example, the platform 4 may have a structure including a first substrate 2 and a second substrate 3 attached to the first substrate 2, or a structure including a compartment plate (not shown) to define one or more chambers containing a fluid and one or more channels through which the fluid flows between the first substrate 2 and the second substrate 3. The first substrate 2 and the second substrate 3 may be formed of a thermoplastic resin.

The joining of the first substrate 2 and the second substrate 3 may be carried out by a variety of methods such as, but not limited to, adhesion using an adhesive or a double-sided adhesive tape, ultrasonic welding, and laser welding.

Hereinafter, microfluidic structures provided within the platform 4 to test samples will be described in more detail.

A sample may be prepared by mixing a fluid and particulate substances with a greater density than that of the fluid. For example, the sample may include biological samples such as, but not limited to, blood, saliva, and urine.

A sample injection chamber 10 may be disposed radially inward of the platform 4, as compared to the locations of other chambers disposed within the platform 4. The sample injection chamber 10 is configured to contain a predetermined amount of a sample, and a sample inlet 8 to inject the sample into the sample injection chamber 10 is formed on the upper surface of the first substrate 2.

The entire sample in which fluid and particulate substances are mixed may be used in a test that uses a fluid. In addition, a sample separation chamber (not shown) may be disposed radially outward of the sample injection chamber 10 to facilitate centrifugally separating the sample by rotating the platform 4. In addition, the sample separation chamber (not shown) may include a space to contain sediment with a relatively high specific gravity and a space to contain substances with relatively low specific gravity.

A dilution chamber 20 to receive the sample may be further disposed within the in the platform 4. The dilution chamber 20 may have a plurality of chambers to respectively store a dilution buffer in different amounts. Volume of the dilution chambers 20 may vary according to the volume of the dilution buffer to be used in the respective test/assay.

An outlet of the dilution chamber 20 may be connected to a distribution channel 23. The distribution channel 23 may include a first portion 21 extending outwardly in a radial direction of the platform 4 from the outlet of the dilution chamber 20 and a second portion 22 extending along the circumferential direction from an external end of the first portion 21. One end of the second portion 22 may be connected to an air vent (not shown). The air vent (not shown) may be disposed such that a sample does not leak when the sample is transferred from the dilution chamber 20 to the distribution channel 23 by centrifugal force.

A reaction chamber group 30 may be disposed radially outward of the dilution chamber 20. If a plurality of dilution chambers 20 are disposed in the platform 4, a plurality of reaction chamber groups 30 may be disposed in the platform 4, each group corresponding to each of the dilution chambers 20.

Each reaction chamber group 30 may include one or more reaction chambers 31. The reaction chamber 31 is connected to the corresponding dilution chamber 20 via the distribution channel 23, thereby distributing the dilution buffer. In an exemplary embodiment, each of the reaction chamber groups 30 may include one reaction chamber 31 in the simplest configuration.

The reaction chamber 31 may be a sealed chamber. A sealed chamber indicates that the reaction chamber 31 does not include a vent for exhaust. Various types of reagents or a reagent of various concentrations which participate in optically detectable reactions with a diluted sample may be previously injected into the one or more reaction chambers 31. Examples of the optically detectable reaction may be variations in fluorescent light emission or absorbance. However, use of the reaction chamber 31 is not limited thereto.

In another exemplary embodiment, the one or more reaction chambers 31 may be a chamber with a vent.

If a plurality of the reaction chamber groups 30 are disposed in the platform 4, reagents suitable for reaction with a diluted sample may be respectively stored in the one or more reaction chambers 31 belonging to the same reaction chamber group 30.

For example, reagents such as triglycerides (TRIG), total cholesterol (Chol), glucose (GLU), and blood urea nitrogen (BUN), which may be involved in a reaction under the condition that the dilution ratio of the dilution buffer/sample is 100:1, may be stored in a first reaction chamber group. Reagents such as direct bilirubin (DBIL), total bilirubin (TBIL), and gamma glutamyl transferase (GGT), which may be involved in reaction under the condition that the dilution ratio of the dilution buffer/sample is 20:1, may be stored in a second reaction chamber group.

That is, since a diluted sample having a different dilution ratio from that of the first reaction chamber group is supplied to one or more reaction chambers of the second reaction chamber group from the corresponding second dilution chamber, reagents suitable for the diluted sample may be stored in each of the reaction chambers of the reaction chamber group.

The reaction chambers 31 may have the same capacity. However, the embodiments described herein are not limited thereto. The capacities of each of the reaction chambers 31 may vary when different amounts of the diluted sample or the reagent are required according to test items.

One or more reaction chambers 31 are connected to the second portion 22 of the distribution channel 23 via a first inlet channel 24.

In addition, in order to ensure reliability of a test performed using the microfluidic device 1, a quality check (QC) may be necessary to confirm that the test is performed in the microfluidic device 1 as designed. As such, the microfluidic device 1 may include a quality check chamber 40 for the QC. . The quality check chamber 40 may be disposed at an end of the distribution channel 23. In addition, the quality check chamber 40 is connected to the distribution channel 23 via a second inlet channel 25. Thus, the sample mixture fills the reaction chambers 31 closest to the dilution chamber 20, first. Then, the sample mixture fills the quality check chamber 40. Thus, it can be seen that all reaction chambers 31 are filled with the sample mixture by checking whether the sample mixture fills the quality check chamber 40. In addition, a vent channel 26 through which air is exhausted may be formed at one side of the quality check chamber 40. The fluid may be transferred to the quality check chamber 40 when air in the quality check chamber 40 is exhausted via the vent channel 26. The vent channel 26 may be formed extending in a radial direction toward the center of the platform 4.

The quality check chamber 40 may include a plurality of containers 41 and 42 which will be described later.

Valves (not shown) may be disposed in channels connecting the chambers. Various types of valves may be used. The valve may be passively opened when a pressure with a predetermined level or higher is applied thereto, and may be, for example, a capillary tube valve. The valve may also be actively operated when power or energy is supplied from the outside through driving signals.

The platform 4 may further include a bar code unit. The bar code unit may store various information such as the date of manufacture of the microfluidic device 1 and information regarding expiration date.

The bar code unit may be a one-dimensional bar code or any of various types of bar codes to store a large amount of information, for example, a matrix code such as a two-dimensional bar code.

The bar code unit may be replaced by holograms, RFID tags, memory chips, and the like capable of storing information. In addition, when a storage medium for reading and writing information, such as a memory chip, is used instead of the bar code unit, sample test results, patient information, the date of blood sampling, the date and time of test, and performance of the test may be stored in addition to identification information.

In addition, a groove positioning unit 6 may be formed at a side surface 7 of the platform 4 of the microfluidic device 1 to set the reference position of the microfluidic device 1.

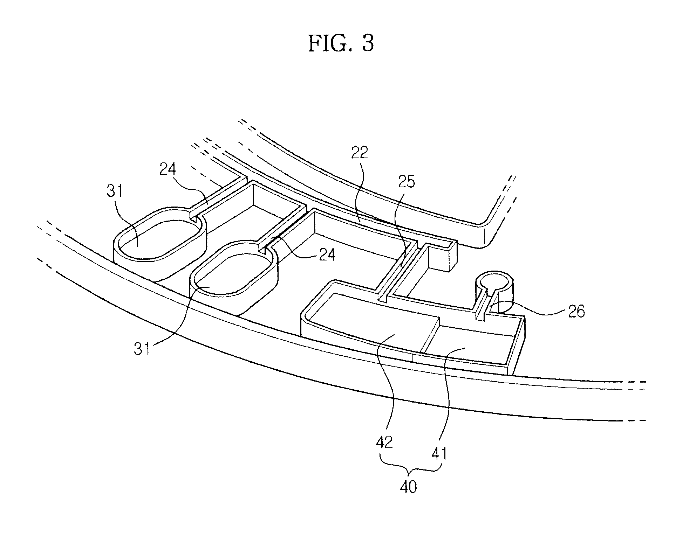

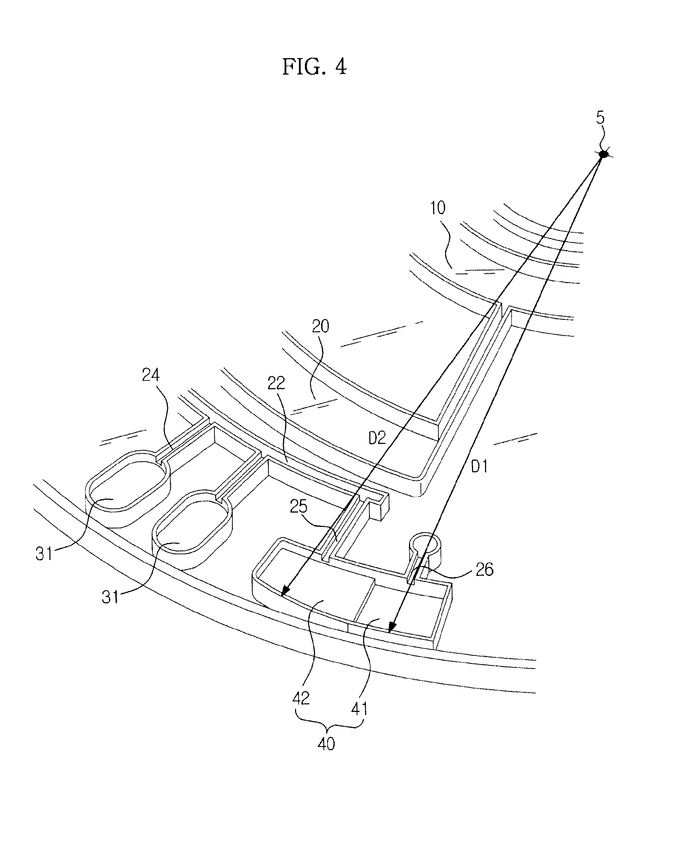

FIG. 3 is an enlarged perspective view of various components of the microfluidic device 1 according to an exemplary embodiment. FIG. 4 is a view of the microfluidic device 1 of an exemplary embodiment illustrating the relationship between quality check chamber 40 and the central axis 5 of the platform 4.

Referring to FIGS. 3 and 4, the quality check chamber 40 of the microfluidic device 1 according to the current exemplary embodiment may include a plurality of containers 41 and 42. Hereinafter, the quality check chamber 40 will be described, but the embodiments described herein are not limited thereto.

The quality check chamber 40 includes a plurality of containers that include a first container 41 and a second container 42. The bottom surfaces of containers 41 and 42 are at different heights relative to each other. In an exemplary embodiment, the bottom surface of the first container 41 is lower than the bottom surface of the second container 42 within the platform 4. In other words, a depth of the first container 41 is greater than a depth of the second container 42. The containers 41 and 42 may therefore have different cross-sectional areas. Thus, if the same amount of a fluid is injected into quality check chamber 40, a height of the fluid contained in the first container 41 is different from that of the fluid contained in the second container 42. In other words, in a top view of the microfluidic device 1, the cross-sectional areas of the first container 41 and the second container 42 are different.

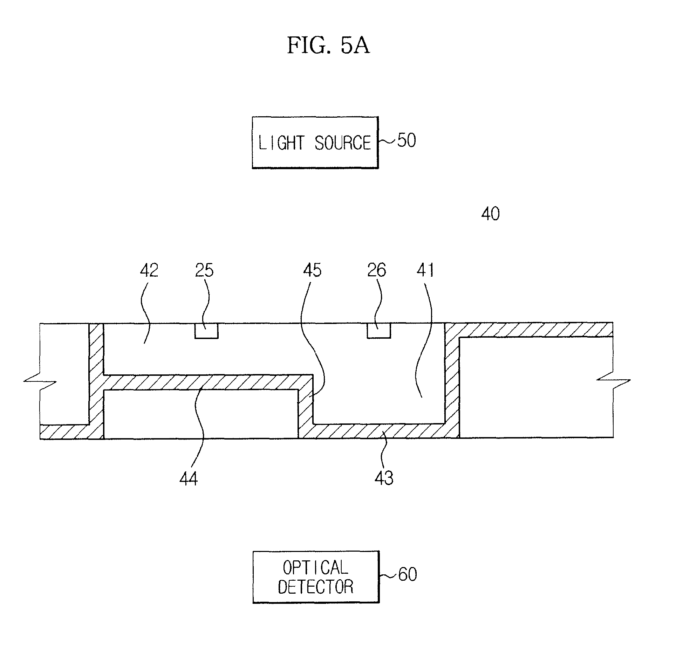

As shown in FIG. 5A, the bottom 44 of the second container 42 is partially grooved to form the first container 41. That is, a portion of the bottom 44 of the second container 42 has a step difference 45, thereby forming the first container 41.

As discussed above, the quality check chamber 40 includes a plurality of containers including the first container 41 and the second container 42 with different cross-sectional areas. Thus, if a fluid is contained in the quality check chamber 40, the height of the fluid is different from that of a fluid contained in a quality check chamber including only one container.

According to Lambert-Beer's law, the relation among absorbance, thickness of a fluid, and concentration of the fluid satisfies the following equation. When A is absorbance, c is a concentration of the fluid, b is a thickness of the fluid, ε is an extinction coefficient, 10 is an intensity of light before passing through the fluid, and I is an intensity of light after passing through the fluid, the relation satisfies the following equation.

$$\text{Absorbance } (A) = \log 10(I0/I) = \varepsilon bc$$

The absorbance (A) may be measured by the optical detector 60. Since the extinction coefficient (ε) is a unique property of a material, and the concentration (c) is a known value, the height of the fluid may be obtained from the absorbance (A). Thus, a volume of the fluid may be calculated. Dyes or fluorescent nanoparticles to increase absorbance may be added to one or more reagents injected to perform an accurate quality check.

Thus, quantitative injection of the reagent used in the microfluidic device 1 may be tested. The height of the reagent contained in the quality check chamber 40 may be measured by measuring absorbance using the optical detector 60 to detect optical energy from a light source 50. Based on the height of the reagent contained in the quality check chamber 40, the flow of the reagent in the microfluidic device 1 and operation of valves (not shown) may be checked. Thus, the quality check may be performed using only the quality check chamber without using a metering chamber and a residual sample chamber, which are conventionally used for quality check operations.

In addition, in a conventional test, when a plurality of reagents having the same concentration and the same amount are used, the concentration of each of the reagents decreases by 1/2, but the thickness of each of the reagents doubles. Thus, the absorbance (A) thereof is the same as that measured when using a single reagent. As such, the number of quality check detection units should need to be the same as that of the reagents in conventional tests.

As discussed above, the quality check chamber 40 according to the current exemplary embodiment includes a plurality of containers 41 and 42, and the cross-sectional areas of the containers 41 and 42 are different from each other. Thus, when a plurality of reagents having the same concentration and the same content are injected into the containers 41 and 42, the thicknesses of the reagents are different from each other. Thus, the absorbance (A) is not the same as that obtained when using only one reagent.

As shown in FIG. 4, a distance between the first container 41 and the central axis 5 of the platform 4 may be greater than a distance between the second container 42 and the central axis 5 of the platform 4. If the distance between the first container 41 and the central axis 5 of the platform 4 is defined as D1, and the distance between the second container 42 and the central axis 5 of the platform 4 is defined as D2, D1 > D2. Thus, the first container 41 is disposed farther than the second container 42 from the central axis 5 of the platform 4. Due to this configuration, when a fluid flows due to centrifugal force while the platform 4 rotates, the first container 41 is filled first. In a top view of the microfluidic device 1, the cross-sectional area of the second container 42 may be greater than that of the first container 41. Since the cross-sectional area of the second container 42 is greater than that of the first container 41, the first container 41 and the second container 42 do not overlap each other in the measurement of absorbance performed by irradiating optical energy thereon, so that the absorbance may be more accurately detected.

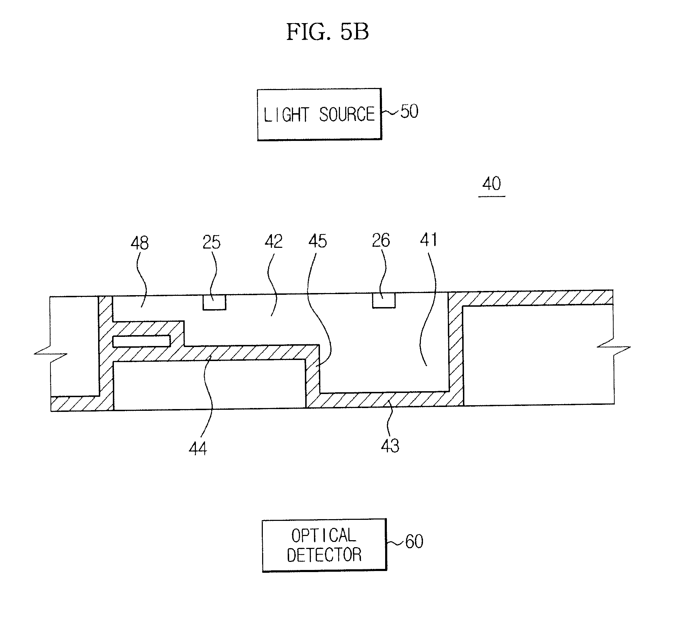

Within the quality check chamber 40, the plurality of the containers may further include another container in addition to the first container 41 and the second container 42. For example, as shown in FIG. 5B, a third container 48 having a cross-sectional area different from that of the second container 42 may be disposed at an upper portion of the second container 42. The bottom surface of the second container 42 may be disposed lower than the bottom surface of the third container 48, and the bottom surface of the first container 41 may be disposed lower than the bottom surface of the second container 42. The second container 42 may have a step difference at the center of the third container 48.

In this case, the first container 41 is disposed at the farthest position from the central axis 5 of the platform 4, and the third container 48 is disposed at the closest position from the central axis 5 of the platform 4. Accordingly, the fluid is filled in the order of the first container 41, the second container 42, and the third container 48.

In addition, the cross-sectional areas of the containers may increase in the order of the third container 48, the second container 42, and the first container 41. In this case, the detection sensitivity of absorbance may be increased.

A quality check chamber 40 including the third container 48 may be used in a microfluidic device using three types of reagents. For performing a quality check, a single quality check chamber may be used.

Accordingly, the containers contained within the quality check chamber 40 may further include another container with different cross-sectional area in addition to the first container 41 and the second container 42.

FIGS. 5A and 5B are cross-sectional views showing a quality check chamber 40 of a microfluidic device 1 according to exemplary embodiments taken along line A-A as shown in Figure 2.

According to the exemplary embodiment shown in FIG. 5A, the microfluidic device 1 is disposed between the light source 50 and the optical detector 60. Accordingly, optical energy irradiated from the light source 50 passes through the microfluidic device 1 including the quality check chamber 40 and is received by the optical detector 60 to be used to measure absorbance of a fluid contained in the quality check chamber 40.

The light source 50 is a light source that flashes at a predetermined wavelength. Exemplary light sources include, but are not limited to, a semiconductor light emitting device such as a light emitting diode (LED) and a laser diode (LD), and a gas discharge lamp such as a halogen lamp and a Xenon lamp.

The optical detector 60 generates electric signals according to the intensity of incident light, and may be a depletion layer photo diode, an avalanche photo diode (APD), or a photomultiplier tube (PMT).

The bottom surface 44 of the second container 42 at the boundary between the first container 41 and the second container 42 may be parallel to the bottom surface 43 of the first container 41. However, the exemplary embodiments described herein are not limited thereto.

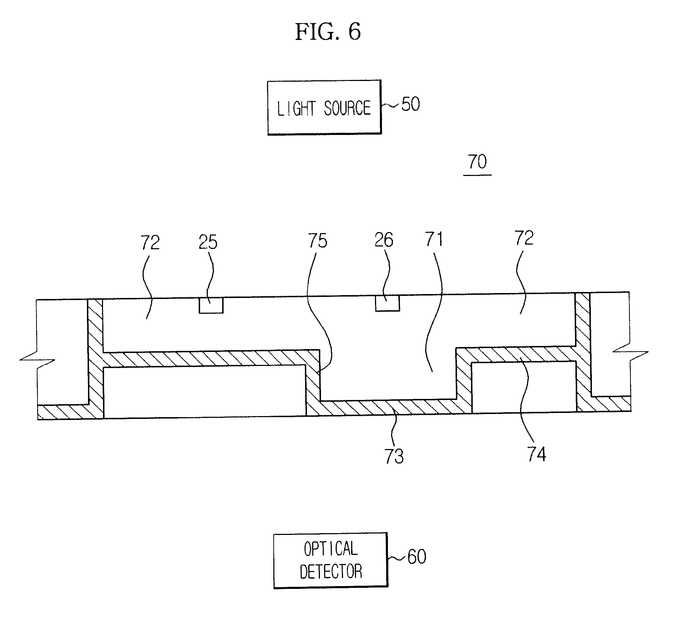

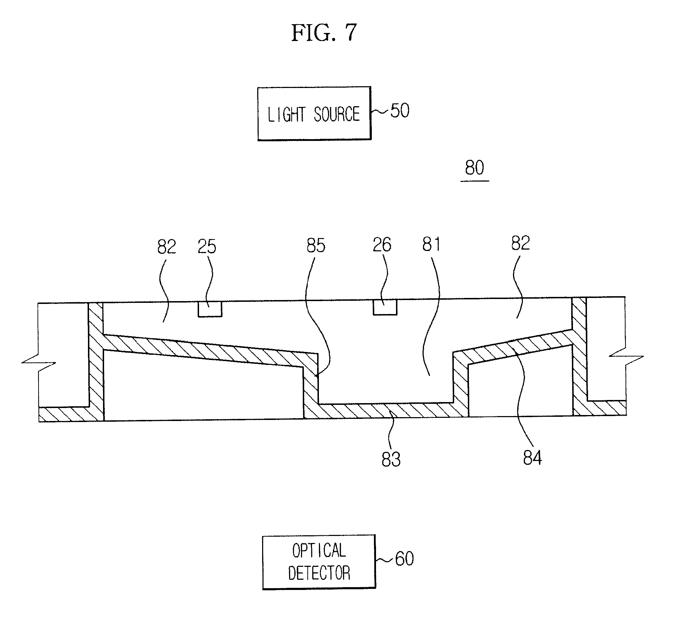

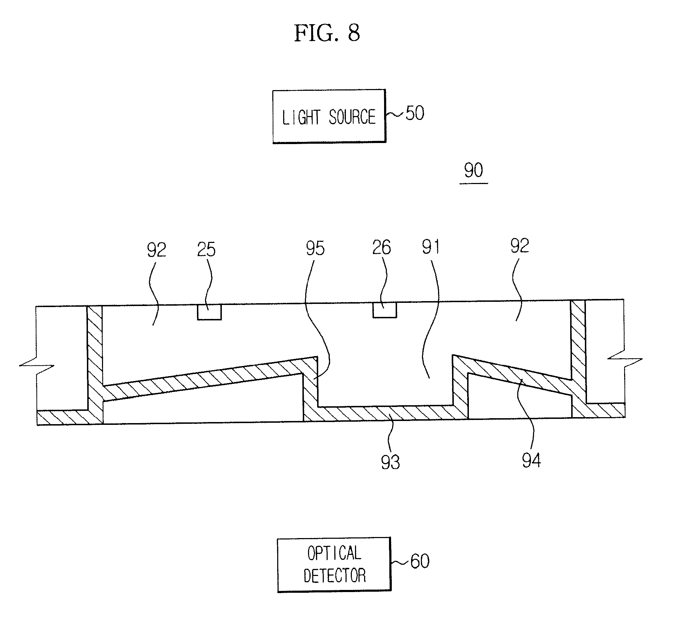

FIG. 6 is a cross-sectional view showing a quality check chamber of a microfluidic device according to another exemplary embodiment taken along line A-A. FIG. 7 is a cross-sectional view showing a quality check chamber of a microfluidic device according to another exemplary embodiment taken along line A-A. FIG. 8 is a cross-sectional view showing a quality check chamber of a microfluidic device according to another exemplary embodiment taken along line A-A.

According to the exemplary embodiment shown in FIG. 6, a first container 71 is formed by grooving a central region of the bottom surface of a second container 72 to form a step difference. That is, the first container 71 is formed at the central region, and the second container 72 is formed at both sides of the first container 71. Accordingly, a fluid may be filled in the first container 71 more efficiently than a structure in which the first container 71 is disposed at an end of the second container 72.

According to the exemplary embodiment shown in FIG. 7, bottom surface 84 of second container 82 is inclined upward from the boundaries between a first container 81 and the second container 82 to the ends (i.e., outer edges) of the second container 82. That is, the bottom surface 84 of the second container 82 slopes upward and outward from the center of the quality check chamber 80.

Since the bottom surface 84 of the second container 82 is inclined, the flow of a fluid injected into the quality check chamber 80 may be controlled.

According to the exemplary embodiment shown in FIG. 8, bottom surfaces 94 of second container 92 decline from the boundaries between a first container 91 and the second container 92 to the ends (i.e., outer edges) of the second container 92. Accordingly, the flow of a fluid injected into the quality check chamber 90 may be controlled.

As shown in FIGS. 7 and 8, in the quality check chambers 80 and 90, including a plurality of containers 81, 82, 91, and 92, the bottoms 84 and 94 of the second containers 82 and 92 may be inclined or declined surfaces with predetermined angles from the boundaries between the first containers 81 and 91 and the second containers 82 and 92 to the ends (i.e., outer edges) of the second containers 82 and 92. Accordingly, flow of fluids may be controlled in the quality check chambers 80 and 90 according to the platform 4 and properties of the fluids.

As is apparent from the above description, errors of tests performed using the microfluidic device may be detected using a single chamber. Thus, even when a plurality of reagents are used, test errors may be detected using a single chamber, and thus the design of the microfluidic device may be simplified.

Although exemplary embodiments have been shown and described, it should be appreciated by those skilled in the art that changes may be made in these embodiments without departing from the principles and spirit of the inventive concept, the scope of which is defined in the claims and their equivalents.

## Claims

1. A microfluidic device comprising:

    a platform;
    one or more chambers disposed in the platform to contain a fluid; and
    one or more channels connecting the one or more chambers,
    wherein at least one of the chambers comprises a a first container and a second container,
    wherein a depth of the first container is greater than a depth of the second container, and
    wherein a cross-sectional area of the first container is different from a cross-sectional area of the second container.

2. The microfluidic device of claim 1, wherein a distance between the first container and a central axis of the platform is greater than a distance between the second container and the central axis of the platform.

3. The microfluidic device of claim 1, wherein when a same amount of fluid is injected into the plurality of containers, a height of the fluid in the first container is different from a height of the fluid in the second container.

4. The microfluidic device of claim 1, wherein a boundary between the first container and the second container is parallel to a bottom surface of the first container.

5. The microfluidic device of claim 1, wherein a boundary between the first container and the second container comprises an inclined surface sloping upward from the first container toward an outer edge of the second container.

6. The microfluidic device of claim 1, wherein a boundary between the first container and the second container comprises a declined surface sloping downward from the first container toward an outer edge of the second container.

7. The microfluidic device of claim 1, wherein the one or more chambers comprises a sample injection chamber to inject a fluid, one or more reaction chambers in which reactions of the fluid occur, and a quality check chamber to confirm whether a test is performed as designed.

8. The microfluidic device of claim 7, wherein the sample injection chamber is disposed within the platform radially inward from the reaction chamber and the quality check chamber.

9. The microfluidic device of claim 1, wherein a cross-sectional area of the second container is greater than a cross-sectional area of the first container.

10. The microfluidic device of claim 1, wherein a step difference is formed at the center of the chambers to form the first container and the second container.

11. The microfluidic device of claim 1, wherein the plurality of containers further comprise a third container, and the depth of the second container is greater than a depth of the third container.

12. The microfluidic device of claim 11, wherein a cross-sectional area of the third container is different from a cross-sectional area of the second container, such that when the same amount of fluid is injected into the containers, a height of the fluid in the third container is different from a height of the fluid in the second container.

13. The microfluidic device of claim 12, wherein the second container is formed by forming a step difference at the center of the third container, and the first container is formed by forming a step difference at the center of the second container.

14. The microfluidic device of claim 1, wherein the platform is rotatable.

FIG. 1

FIG. 2

FIG. 3

FIG. 4

## FIG. 5A

LIGHT SOURCE ~50

40

42    25    45    26    41

44    43

OPTICAL
DETECTOR ~60

# FIG. 5B

LIGHT SOURCE ~50

40

48    25    42  45    26    41

OPTICAL
DETECTOR ~60

FIG. 6

LIGHT SOURCE ~50

<u>70</u>

72    25        75        26    71        72

73        74

OPTICAL
DETECTOR ~60

# FIG. 7

LIGHT SOURCE ~50

80

82  25        85        26  81        82

OPTICAL DETECTOR ~60

83        84

# FIG. 8

LIGHT SOURCE ~50

<u>90</u>

92  25      95   26  91      92

93

94

OPTICAL
DETECTOR ~60

Europäisches
Patentamt

European
Patent Office

Office européen
des brevets

# EUROPEAN SEARCH REPORT

Application Number

EP 12 19 5366

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/129583 A1 (BEDINGHAM WILLIAM [US] ET AL) 16 June 2005 (2005-06-16) * paragraphs [0023] - [0066]; figures * ----- | 1-14 | INV. B01L3/00 |
| X | US 2010/009431 A1 (CHO YOONKYOUNG [KR] ET AL) 14 January 2010 (2010-01-14) * the whole document * ----- | 1-14 | |
| X | WO 98/43739 A2 (BIOSITE DIAGNOSTICS INC [US]) 8 October 1998 (1998-10-08) * figure 11; example 22 * ----- | 1-14 | |
| A | US 2010/151561 A1 (RICHTER ANDREAS [DE] ET AL) 17 June 2010 (2010-06-17) * abstract; figure 8 * ----- | 1-14 | |
| | | | TECHNICAL FIELDS SEARCHED (IPC) |
| | | | B01L |

The present search report has been drawn up for all claims

| Place of search | Date of completion of the search | Examiner |
|---|---|---|
| Munich | 30 April 2013 | Smith-Hewitt, Laura |

EPO FORM 1503 03.82 (P04C01)

**ANNEX TO THE EUROPEAN SEARCH REPORT
ON EUROPEAN PATENT APPLICATION NO.**                    EP 12 19 5366

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2013

| Patent document cited in search report | | Publication date | Patent family member(s) | | | Publication date |
|---|---|---|---|---|---|---|
| US 2005129583 | A1 | 16-06-2005 | AT | 399054 | T | 15-07-2008 |
| | | | AU | 2004305486 | A1 | 07-07-2005 |
| | | | CA | 2548414 | A1 | 07-07-2005 |
| | | | CN | 1890018 | A | 03-01-2007 |
| | | | EP | 1699548 | A1 | 13-09-2006 |
| | | | JP | 4988354 | B2 | 01-08-2012 |
| | | | JP | 2007513757 | A | 31-05-2007 |
| | | | US | 2005129583 | A1 | 16-06-2005 |
| | | | US | 2011027904 | A1 | 03-02-2011 |
| | | | WO | 2005061084 | A1 | 07-07-2005 |
| US 2010009431 | A1 | 14-01-2010 | US | 2010009431 | A1 | 14-01-2010 |
| | | | WO | 2010005197 | A2 | 14-01-2010 |
| WO 9843739 | A2 | 08-10-1998 | AT | 242054 | T | 15-06-2003 |
| | | | AU | 6579098 | A | 22-10-1998 |
| | | | CA | 2284801 | A1 | 08-10-1998 |
| | | | DE | 69815365 | D1 | 10-07-2003 |
| | | | DE | 69815365 | T2 | 19-08-2004 |
| | | | EP | 1019193 | A2 | 19-07-2000 |
| | | | JP | 3531941 | B2 | 31-05-2004 |
| | | | JP | 2001526778 | A | 18-12-2001 |
| | | | US | 6156270 | A | 05-12-2000 |
| | | | US | 6271040 | B1 | 07-08-2001 |
| | | | WO | 9843739 | A2 | 08-10-1998 |
| US 2010151561 | A1 | 17-06-2010 | DE | 102006051535 | A1 | 18-12-2008 |
| | | | DE | 112007003160 | A5 | 24-09-2009 |
| | | | EP | 2094387 | A2 | 02-09-2009 |
| | | | US | 2010151561 | A1 | 17-06-2010 |
| | | | WO | 2008049413 | A2 | 02-05-2008 |

EPO FORM P0459

For more details about this annex : see Official Journal of the European Patent Office, No. 12/82